# EUROPÄISCHE PATENTANMELDUNG

(11) **EP 2 266 688 A2**
(43) Veröffentlichungstag der Anmeldung: **29.12.2010**
(21) Anmeldenummer: 10178321.5
(22) Anmeldetag: 04.10.2007
(51) Int. Cl.: B01F 13/10, B01F 7/20, B01F 5/06, B01F 15/02, A45D 40/00, A45D 44/00

(54) **Kosmetische Zubereitung**

(30) Priorität: 13.10.2006 DE 102006049056
(62) Teilanmeldung aus: 07818671.5
(71) Anmelder: Beiersdorf AG, 20253 Hamburg (DE)
(72) Erfinder: Hoffmann, Dr. Nils, 22303, Hamburg (DE); Weyland, Silke, 67551, Worms (DE); Hofmann, Andreas, 22303, Hamburg (DE)

(57) **Zusammenfassung**

Die Erfindung bezieht sich auf eine Vorrichtung zur Zubereitung von Kosmetika und ein entsprechendes Verfahren zur Zubereitung von Kosmetika unter Verwendung der Vorrichtung.

## Beschreibung

Die Erfindung bezieht sich auf eine Vorrichtung zur Zubereitung von Kosmetika und ein entsprechendes Verfahren zur Zubereitung von Kosmetika unter Verwendung der Vorrichtung.

Aus dem Bereich der Lebens- und Genussmittel ist es bekannt ein Lebens-/Genussmittel kurz vor seinem Verzehr zuzubereiten. Eines der bekanntesten Beispiele ist hierbei die Zubereitung von Kaffeeextrakten aus getrockneten und gerösteten Samen des Kaffeestrauches (*Coffea arabica* (Arabica-Kaffee) und *Coffea canephora* (Robusta-Kaffee). Hierzu werden die gemahlenen Kaffeebohnen mit heißem, nahezu kochendem Wasser überbrüht. Das dadurch erhaltene Filtrat kann anschließend getrunken werden.

Auch andere Getränke lassen sich inzwischen kurz vor dem Verzehr frisch zubereiten. Z.B. ist es unter Einsatz einer Begasungsvorrichtung möglich, aus Leitungswasser und Kohlendioxid frisches Tafelwasser zu schaffen. Dieses kann, je nach Bedarf und Geschmack, zusätzlich mit Aroma-/Frucht-/Vitamin-/Mineralstoff-Konzentraten verfeinert werden, so dass verschiedenste Brausen (gashaltige, sprudelnde Tafelwässer) entstehen. Das, was als Herstellungsprozedur zuvor in Fabriken stattfand, wird so im Kleinmaßstab in die heimische Küche verlagert. Dies hat den Vorteil, dass die Zubereitung bedarfsgerecht erfolgen kann und durch den Einsatz von Konzentraten (Aroma, Mineralien) keine großen Lagerkapazitäten in der Heimstatt benötigt werden. Auch aus logistischer Sicht beinhaltet die Eigenherstellung Vorteile, da keine großen Flüssigkeitsmengen mehr transportiert werden müssen.

Außerhalb des Nahrungsmittelbereiches finden sich im täglichen Lebensbedarf noch keine solch effektiven Anwendungen und Verfahren.

Der Mensch an sich findet sein Auskommen jedoch nicht nur durch die Zufuhr von Nahrung, sondern es werden viele weitere Dinge benötigt, um nicht nur zu überleben, sondern auch ein glückliches Leben führen zu können.

Zu diesen Annehmlichkeiten, die aus dem Leben eines Menschen nicht mehr wegzudenken sind, gehören auch Produkte, die zur Pflege des Körpers dienen, insbesondere sind das Kosmetika, Wasch- und Reinigungszubereitungen, Pflegezubereitungen sowie Dekorative Kosmetik.

Ganz besonders bei den Pflegezubereitungen gibt es je nach Applikationsort (Handkrem, Augenkrem, Bodylotion...) und Applikationszeitpunkt (Tageskrem vs. Nachtkrem) eine Vielzahl von Zubereitungen auf dem Markt, die sich meist nur in wenigen Zubereitungsbestandteilen unterscheiden. Will der Mensch also das Beste für seinen Körper, benötigt er eine ganze Reihe von Produkten um alle Anwendungsbereiche abzudecken, insbesondere da nicht alle Produkte täglich benötigt werden. Lichtschutzzubereitungen zum Beispiel müssen nicht an bewölkten Tagen oder in der Nacht angewendet werden.

Nachteilig ist derzeit, dass alle Produkte einzeln erworben werden müssen und im heimischen Badezimmer- dort liegt nach Umfragen der hauptsächliche Lager- und Anwendungsort - sehr viel Platz beanspruchen. Insbesondere ist durch die unterschiedlichen Verpackungseinheiten die benötigte Staufläche sehr groß, da sich Tuben, Tiegel oder Flaschen nicht gemeinsam stapeln lassen. Ein weiterer Nachteil, der in der letzten Zeit immer stärker in den Vordergrund tritt, ist, dass spezifische Wirkstoffe, die den Pflegeprodukten zugesetzt wurden, in zubereiteter Form nur kurzzeitig Stabilität bzw. Wirkung zeigen. Um marktfähige Produkte in den Handel bringen zu können, muss daher tief in die Trickkiste der Formulierungstechnik gegriffen werden. Insbesondere die Komplexierung von Wirkstoffen oder der Einsatz von gasdiffusionsdichten Laminatbehältnissen wirken sich auf den Herstellungspreis der Produkte negativ aus, so dass dieser entsprechend steigt.

Ein anderer Aspekt ist die Keimanfälligkeit kosmetischer Zubereitungen. Für die bei der Entnahme mit den Fingern eingebrachten Keime stellen die wasserhaltigen Zubereitungen eine ideale Wachstumsgrundlage dar. Die kosmetische Industrie versucht daher mit hohem Aufwand den Eintrag von Keimen zu verhindern - z.B. durch Spendersysteme - bzw. die Vermehrung nach der Kontamination stark einzuschränken - z.B. durch den Einsatz von Konservierungsstoffen.

Ein Ausweg ist die Herstellung von kleinen Mengen kosmetischer Zubereitungen direkt vor der Anwendung der Zubereitung. Ansätze gab es hier schon einige, wie z.B. in der deutschen Fernsehsendung 'Hobbythek' demonstriert. Jedoch stellen diese eher eine Spielerei mit Lerneffekt dar, als eine nachhaltige, ständig anzuwendende Lösung.

Wünschenswert wäre es daher, eine Vorrichtung und ein Verfahren zur Verfügung zu stellen, mit der/dem es möglich ist, Zubereitungen die der Pflege des Körpers dienen, kurzfristig und vor Ort (beim Anwender zu Hause) herzustellen, wobei diese Anwendungszubereitungen aus einer oder mehreren Grundzubereitung(en) unter Zugabe von Wirkstoffkonzentraten in den zur einmaligen Anwendung üblichen Mengen zubereitet werden.

Gelöst wird diese Aufgabe durch eine Vorrichtung, aufweisend
- ein oder mehrere Behältnisse für ein oder mehrere Grundzubereitungen,
- eine Dosiereinrichtung,
- mindestens eine elektrisch betriebenen Pumpe,
- eine Mischkammer mit einer verschließbaren Öffnung,
- eine Mischereinheit und
- eine Ausgabeöffnung,
**dadurch gekennzeichnet, dass** die Grundzubereitung(en) aus den Vorratsbehältniss(en) mit Hilfe der elektrischen Pumpe(n) in die Mischkammer transportiert werden, wobei die Menge an Grundzubereitung über eine Dosiereinrichtung gesteuert wird und wobei die Mischkammer eine verschließbare Öffnung zur Umgebung aufweist, durch die es möglich ist, händisch zusätzliche Komponenten (wie z.B. Wirkstoffe), die der Grundzubereitung zugemischt werden sollen, zuzudosieren.

Erfindungsgemäß lassen sich mit der erfindungsgemäßen Vorrichtung bzw. im erfindungsgemäßen Verfahren verschiedene Grundzubereitungen zur einer kosmetischen Zubereitung kombinieren, wobei das Verhältnis der Grundzubereitungen zueinander durch die Dosiereinrichtung gesteuert bzw. vorgegeben wird und die Menge an zusätzlichen Komponenten durch den Anwender selbst bestimmt wird.

Erfindungsgemäß werden die Grundkomponenten nacheinander oder simultan in die Mischkammer eingespeist. Dazu sind Mischkammer und der oder die Behältnisse für die Grundkomponenten über ein Schlauch-, Rohr- und/oder Kanalsystem miteinander verbunden.

Die Grundzubereitungen werden in Vorratsbehältnissen gelagert. Diese Behältnisse sind erfindungsgemäß einzeln oder in Gruppen austauschbar. Bevorzugt haben die Behältnisse ein Volumen von 10 bis 1000 ml, je nach dem, um welche Art von Grundzubereitung es sich handelt. Ganz besonders vorteilhaft ist es, Behältersysteme einzusetzen, bei denen das entnommene Volumen nicht durch Umgebungsluft ersetzt wird, insbesondere eignen sich Schleppkolbenbehälter, flexible Beutel (z.B. Folienbeutel, Bag-in-Can-Packmittel) oder Sachets zur Verwendung als erfindungsgemäßes Vorratsbehältnis.

Die Mischkammer weist eine verschließbare Öffnung auf, durch die sich individuell zusätzliche Komponenten in die Grundkomponenten eintragen lassen. Die zusätzlichen Komponenten können in fester oder flüssiger Form vorliegen, insbesondere von Vorteil ist es, wenn die zusätzlichen Komponenten eine kompakte Form aufweisen (insbesondere Tabletten oder Kapseln). Die Kapseln oder Tabletten werden beim Mischvorgang zerkleinert oder aufgebrochen und liegen nach erfolgtem Mischvorgang in der Anwendungszubereitung gleichmäßig verteilt vor.

Die Mischkammer muss dazu mindestens eine dynamische Mischereinheit aufweisen, mit dem die Grundkomponenten und die über die verschließbare Öffnung eingebrachten, zusätzlichen Komponenten zu einer homogenen Masse vermischt werden können. Unter Mischereinheit sind erfindungsgemäß rotierende Elemente zu verstehen, die elektrisch oder per Muskelkraft angetrieben werden.

Ganz besonders vorteilhaft ist es, wenn die dynamische Mischvorrichtung ein Homogenisator ist. Homogenisatoren zeichnen sich dadurch aus, dass durch schnell rotierende Flügelräder hohe Scheerkräfte auf die Zubereitungen ausgeübt werden, wodurch z.B. die Bildung von Emulsionen stark begünstigt wird.

Erfindungsgemäß ist es auch, dass die Kapseln vor dem Mischvorgang geöffnet und ausgepresst werden, so dass sich der Inhalt in die vorliegenden Grundzubereitungen ergießt. Leere Kapselhüllen bleiben dabei zurück und können dem Abfall zugeführt werden.

Nachdem die Grundzubereitungen und die zusätzliche Komponente zu einer homogenen Masse (Anwendungszubereitung) vermischt wurden, wird die Anwendungszubereitung aus der Mischkammer ausgetragen. Der Austrag kann entsprechend der Einspeisung diskontinuierlich (Verwendung eines dynamischen Mischers) erfolgen. Hierzu weist die Mischkammer eine entsprechende Auslassöffnung auf. Zwischen Mischkammer und Auslassöffnung kann ein Ventil oder durchflußbegrenzendes Element angeordnet sein.

Da kosmetische Zubereitungen teilweise hohe Viskositäten aufweisen, die einen Austrag mittels Schwerkraft nicht erlauben, ist es auch im Sinne der Erfindung den Austrag aus der Mischkammer durch Gas- oder Kolbendruck zu beschleunigen. Für kosmetische Zubereitungen von kremartiger Konsistenz ist die kontinuierliche Mischung unter Verwendung einer statischen Mischeinrichtung ganz besonders bevorzugt, da hier der Austrag der Anwendungszubereitung durch die nachgeförderten Grundzubereitungen erfolgt.

Die Menge jeder einzelnen Grundkomponente wird über die Dosiereinrichtung gesteuert. Erfindungsgemäß sind unter Dosiereinrichtung alle technischen Maßnahmen zu verstehen, die den Volumenstrom der Grundkomponenten begrenzen oder variieren.

Im einfachsten Fall ist die Dosiereinrichtung eine Anzahl von variabel einstellbaren Durchflußbegrenzern, die, je nach dem welche Anwendungszubereitung aus den Grundzubereitungen hergestellt werden soll, entsprechend von Hand eingestellt werden.

Vorteilhaft erfolgt die Einstellung jedoch elektromechanisch. Die Dosiereinrichtung kann demnach eine Speicher Programmierbare Steuerung (SPS) in Kombination mit steuerbaren Ventilen, Durchflußbegrenzern oder Pumpen enthalten. Insbesondere erfindungsgemäß und vorteilhaft ist es, die Pumpenfördermenge, zum Beispiel über die Drehzahl oder Einschaltzeit der Pumpe, zu variieren. Diese Betriebsweise ist insbesondere bei simultaner Förderung aller Grundkomponenten nötig, um das richtige Verhältnis der Grundkomponenten zueinander einzustellen. Hierzu werden innerhalb der SPS die Mischungsverhältnisse in einer Datenbank abgelegt und sind vom Anwender entsprechend über eine Eingabeeinheit abrufbar.

Erfindungsgemäß ist auch die Steuerung von Ventilen oder Absperrschiebern durch die Dosiereinheit und die Verwendung nur einer Pumpe zur Förderung aller Grundkomponenten.

Erfindungsgemäß kann die oder können die Grundzubereitung(en) einphasige oder mehrphasige Systeme sein. Z.B. ist es möglich, eine kosmetische Zubereitung (Anwendungszubereitung) aus zwei Grundzubereitungen und einer zusätzlichen Komponente, wobei die Grundzubereitung eine Ölphase und die Andere eine wässrige Phase darstellt, mit der erfindungsgemäßen Vorrichtung herzustellen. Je nach dem wie das Verhältnis zwischen Öl- und Wasserphase eingestellt wird, werden Emulsionen mit unterschiedlichen Eigenschaften, wie zum Beispiel Viskosität und/oder Hautgefühl, erhalten.

Es ist ebenfalls erfindungsgemäß, wenn ein oder mehrere Grundzubereitungen bereits mehrphasige Systeme, wie zum Beispiel Öl-in-Wasser- oder Wasser-in-Öl-Emulsionen sind.

Erfindungsgemäß ist es auch noch weitere Grundzubereitungen einzuarbeiten, so dass die fertige Zubereitung (Anwendungszubereitung) mindestens drei Grundzubereitungen enthält.

Erfindungsgemäß lassen sich mit der erfindungsgemäßen Vorrichtung bzw. im erfindungsgemäßen Verfahren zusätzliche Komponenten, insbesondere konzentrierte Zusätze, in die Grundzubereitung oder die Mischung der Grundzubereitungen einarbeiten, wobei unter den Zusätzen im Sinne der Erfindung auch Parfümstoffe, klassische Wirkstoffe, Emulgatoren, Farbstoffe bzw. Pigmente, Lichtschutzfilter, Moisturizer, Peelingpartikel, Tages- und Nachtpflegestoffe, Polymere, Konsistenzgeber, Wasser, Öle, Mineralstoffe, Seren, Pflanzenextrakte, Füllstoffe, Sensorikadditive, Alkohole sowie Selbstbräuner verstanden werden.

Insbesondere die klassischen Wirkstoffe, wie z.B
- Antioxidantien, wie zum Beispiel Ubichinon Q10,
- Pflanzenextrakte, wie zum Beispiel AleoVera,
- körpereigene Polymere, wie zum Beispiel Hyaluronsäure, Creatin,
- Vitamine, wie zum Beispiel Vitamin C oder E, Folsäure,
- Hautaufheller (Whitening-Wirkstoffe), wie zum Beispiel Dioic Acid,
- selbstbräunende Wirkstoffe (Tanning-Wirkstoffe), wie zum Beispiel Dihydroxyaceton (DHA),
- Lichtschutzfilter, wie zum Beispiel Triazine, Hydroxyzimtsäureester, Metalloxide, können so, je nach Anwendungsort und -zeitpunkt, in die kosmetische Zubereitung in optimalen Konzentrationen eingearbeitet werden.

Erfindungsgemäß enthalten die Anwendungszubereitungen zwischen 0,001 und 25 Gew.- % an klassischen Wirkstoffen, bezogen auf das Gesamtgewicht an Zubereitung. Insbesondere vorteilhaft ist es, wenn die Anwendungszubereitung 0,5-5 Gew.-% DHA und/oder 0,1-5% Gew.-% Vitamin C (Ascorbinsäure) und/oder 0,1-5 Gew-% wässriger Anisfruchtextrakt (Bioxilift) und/oder 0,1-1 Gew.-% Creatin und/oder 0,001 -1 % Gew.-% an pflanzlichen Polyphenolen (z.B. Flavonoide, AGR, Catechine) und/oder 0,001-1% Gew.-% Licochalcone und/oder 0,001-0,3 Gew.-% Folsäure und/oder 0,001-0,5 Gew.-% Q10 enthalten, bezogen auf das Gesamtgewicht der Zubereitung.

Durch die geeignete Auswahl des Parfüms, lässt sich so erstmalig der 'Männlich-Weiblich-Unisex-Konflikt', dem die Hersteller von Pflegeprodukten ständig ausgesetzt sind, umgehen und aus einer Grundzubereitung zu Hause das männliche Produkt und das weibliche Produkt einzig und allein durch Variation des Parfüms zubereiten. Die verschiedenen Duftvarianten /-zusätze, wie z.B. süße oder herbe Duftnoten, ermöglichen es, Zubereitungen, die an die Verwendung (Nacht, Tag, Sommer, Winter, Morgens, Abends) angepasst sind, herzustellen.

Spezielle Ausgestaltungen einer erfindungsgemäßen Vorrichtung, können Mittel zum Begasen, Erwärmen oder Abkühlen der Grundzubereitungen bzw. der Ausgabezubereitungen aufweisen. Vorteilhaft ist es, die Erwärmung, Abkühlung und Begasung ebenfalls über die SPS der Dosiereinheit zu steuern.

Eine Erwärmung der Grundkomponenten kann zum Beispiel den Mischvorgang vereinfachen, da mit der Erwärmung eine Viskositätserniedrigung einhergeht. Durch ein anschließendes Abkühlen beim Austrag der Ausgabezubereitung lässt sich die Temperatur schnell auf die Anwendungstemperatur senken.

Ganz besonders Vorteilhaft ist es, die Ausgabezubereitung, je nach dem um welchen Typ kosmetischer Zubereitung es sich handelt, vor dem bzw. beim Austrag abzukühlen oder zu erwärmen. Eine Erwärmung ist sehr einfach durch elektrische Heizwendeln, eine Abkühlung einfach durch Peltier-Elemente zu erreichen.

Bei Gesichtsmasken oder Haarkuren ist es vorteilhaft, die Temperatur über die Raumtemperatur anzuheben, da diese den pflegenden Effekt (Porenöffnung durch Wärme) verstärkt. Bei einer Augenkrem oder Gesichtstonic dagegen ist eine Temperatur unterhalb der Raumtemperatur von Vorteil, da dadurch ein entspannendes und erfrischendes Gefühl entsteht.

Um eine schaumförmige Ausgabezubereitung zu erhalten, kann in der Mischkammer oder im Ausgabekanal eine Begasung erfolgen. Der Gaseintrag kann ebenfalls über die Dosiereinrichtung gesteuert werden. Vorteilhaft werden hierzu insbesondere Sauerstoff, Stickstoff, Lachgas oder Kohlendioxid verwendet.

Erfindungsgemäß vorteilhaft ist eine Begasung mit Sauerstoff, so dass die Anwendungszubereitung ein Mousse ist, insbesondere mit einem Sauerstoffgehalt von bis zu 25 Vol.- % (bezogen auf das Gesamtvolumen der Zubereitung).

Ein weiterer Vorteil der erfinderischen Vorrichtung liegt darin, das auf Konservierungsmittel verzichtet werden kann, da die Ausgabezubereitung zum sofortigen Verbrauch bestimmt ist.

Die Erfindung wird im folgenden anhand der Figuren näher erläutert.

Die Erfindung ist nicht auf das vorstehend Beschriebene und in den Zeichnungen Gezeigte beschränkt, und viele Modifikationen sind denkbar, ohne eine Abweichung vom Umfang der beigefügten Ansprüche darzustellen.

### Bezugszeichen

- 1: Behältnis für Grundzubereitung
- 2: Förderpumpe
- 3: Ventil, Durchflussregler
- 4: Mischkammer
- 6: Mischereinheit, dynamischer Mischer (Rührwerk)
- 7: Ausgabekanal mit Produktauslass
- 8: Verbindung, Kupplung
- 9: Injektor
- 10: Gasreservoir
- 11: Wärmetauscher (Heizeinrichtung und/oder Kühleinrichtung)
- 12: SPS
- 13: verschließbare Mischkammeröffnung
- D: Dosiereinheit

Figur 1 zeigt den schematischen Aufbau der erfindungsgemäßen Vorrichtung (100) (in einer sehr kostengünstigen Variante), enthaltend ein Behältnis für eine Grundzubereitung (1) welches über eine Kupplung (8) mit dem Leitungssystem der Vorrichtung verbunden ist. Aus diesem Behältnis wird mittels der Pumpe (2) die Grundzubereitung in die Mischkammer (4) gefördert. Die Mischkammer trägt in ihrem Innenraum einen dynamischen Mischer (Rührwerk - 6) und eine verschließbare Öffnung (13). Durch diese Öffnung lassen sich zu den Grundzubereitungen sich die zusätzlichen Komponenten händisch hinzufügen. An die Mischkammer schließt sich der Ausgabekanal mit einem Produktauslass (7) an. Die aus dem Behältnissen zu entnehmende Menge an Grundzubereitung wird über die Dosiereinheit (D) geregelt, wobei die Dosiereinheit D eine SPS (12) und eine regelbare Pumpe (2) aufweist. Der Mischer (6) und das Ausgabeventil (3.4) werden ebenfalls über die SPS (12) gesteuert.

Figur 2 zeigt schematisch den Aufbau einer erfindungsgemäßen Vorrichtung (200), enthaltend drei Behältnisse für Grundzubereitungen (1.1, 1.2, 1.3) die jeweils über eine Kupplung (8.1, 8.2, 8.3) mit dem Leitungssystem der Vorrichtung verbunden sind. Die Pumpen speisen die aus den Behältnissen (1.x) entnommene Grundzubreitung in die Mischkammer (4) ein. Die Mischkammer trägt in ihrem Innenraum einen dynamischen Mischer (Rührwerk - 6) und eine verschließbare Öffnung (13). Durch diese Öffnung lassen sich zu den Grundzubereitungen die zusätzlichen Komponenten händisch hinzufügen. An die Mischkammer schließt sich der Ausgabekanal mit einem Produktauslass (7) an. Die auszugebende Zubereitung wird im Ausgabekanal mittels eines Injektors (9) begast, wobei das Gas aus einem Gasreservoir, hier Druckgasflasche (10) entnommen wird. Die in Figur 2 beschriebene Variante der erfindungsgemäßen Vorrichtung ist insbesondere für niederviskose Zubereitungen geeignet, die in Form eines Schaumes (Mousses) angewendet werden.

Figur 3 zeigt schematisch den Aufbau einer erfindungsgemäßen Vorrichtung (300), enthaltend drei Behältnisse für Grundzubereitungen (1.1, 1.2, 1.3) die jeweils fest mit dem Leitungssystem der Vorrichtung verbunden sind. Die aus den Behältnissen zu entnehmende Menge an Grundzubereitung wird über die Dosiereinheit (D) geregelt, wobei die Dosiereinheit D eine SPS (12) und für jede Grundzubereitung einen Durchflussregler (3.1, 3.2, 3.3) aufweist. Die Durchflussregler speisen eine Pumpe (2), die die Grundzubereitungen in die Mischkammer (4) befördern. Die Dosiereinrichtung steuert den Fluss der Grundzubereitungen vorteilhaft so, dass die Pumpe jeweils nur aus einem Behältnis gespeist wird. Die Mischkammer trägt in ihrem Innenraum einen dynamischen Mischer (Rührwerk - 6) und eine verschließbare Öffnung (13). Durch diese Öffnung lassen sich zu den Grundzubereitungen die zusätzlichen Komponenten händisch hinzufügen. An die Mischkammer schließt sich der Ausgabekanal mit einem Produktauslass (7) an. Der Austrag der Anwendungszubereitung wir über ein im Ausgabekanal befindliches Ventil (3.4) gesteuert. Besonders vorteilhaft ist es, wenn die Dosiereinrichtung die Zugabe der Grundzubereitungen so steuert, dass ein kreisförmiger Ventilzyklus entsteht, das bedeutet, wenn die als erstes in die Mischkammer gepumpte Grundzubereitung zu Beginn nur in einer Teilmenge in die Mischkammer gefördert wird und die restliche Menge am Ende des Zugabezyklus zugegeben wird. Dadurch verweilt zwischen zwei Mischvorgängen immer dieselbe Grundzubereitung in der Pumpe und im Großteil des Leitungsnetzes.

## Patentansprüche

1. Kosmetische Zubereitung, hergestellt mit einer Vorrichtung aufweisend
- ein oder mehrere Behältnisse (1) für ein oder mehrere Grundzubereitungen,
- eine Dosiereinrichtung (D),
- mindestens eine elektrisch betriebene Pumpe (2),
- eine Mischkammer (4),
- eine Mischereinheit (5, 6) und ein Volumen von 0,1 bis 50 ml, insbesondere von 0,5 bis 15ml,
- eine Ausgabeöffnung (7),
- wobei die Mischkammer eine verschließbare Öffnung (13) zur Umgebung aufweist, durch die händisch ein oder mehrere zusätzliche Komponenten in die Mischkammer verbracht werden können und ein Volumen von 0,1 bis 50 ml, insbesondere von 0,5 bis 15 ml aufweist, **dadurch gekennzeichnet, dass**
- die Grundzubereitungen aus den Behältnissen (1) mit Hilfe der elektrischen Pumpe(n) (2) in die Mischkammer (4) transportiert werden,
- die Menge an Grundzubereitung über eine Dosiereinrichtung (D) gesteuert wird,
- die Grundzubereitungen und die zusätzlichen Komponenten (Wirkstoffe) innerhalb der Mischkammer durch die Mischereinheit homogen zur kosmetischen Zubereitung vermengt werden und
- die kosmetische Zubereitung durch die Ausgabeöffnung ausgegeben wird.

2. Kosmetische Zubereitung nach Anspruch 1, **dadurch gekennzeichnet, dass** die Mischereinheit der Vorrichtung ein statischer Mischer ist.

3. Kosmetische Zubereitung nach Anspruch 1, **dadurch gekennzeichnet, dass** die Mischereinheit der Vorrichtung ein dynamischer Mischer ist, insbesondere ein Rührwerk oder Homogenisator.

4. Kosmetische Zubereitung nach mindestens einem der vorhergehenden Ansprüche, **dadurch gekennzeichnet, dass** die Vorrichtung eine Speicher-Programmierbare-Steuerung (SPS) enthält, die zumindest die Menge der einzusetzenden Grundzubereitungen regelt.

5. Kosmetische Zubereitung nach mindestens einem der vorhergehenden Ansprüche, **dadurch gekennzeichnet, dass** die Vorrichtung eine Dosiereinrichtung enthaltend eine Speicher-Programmierbare-Steuerung und ein oder mehrere steuerbare Ventile bzw. Durchflussbegrenzer oder regelbare Pumpen enthält.

6. Kosmetische Zubereitung nach mindestens einem der vorhergehenden Ansprüche, **dadurch gekennzeichnet, dass** die Behältnisse (1) der Vorrichtung ein Volumen von 10 bis 1000 ml, insbesondere von 29 bis 569 ml aufweisen.

7. Kosmetische Zubereitung nach einem der vorhergehenden Ansprüche, **dadurch gekennzeichnet, dass** die Vorrichtung Mittel zum Begasen, Erwärmen oder Abkühlen der Grundzubereitungen bzw. der Ausgabezubereitungen aufweist.

8. Kosmetische Zubereitung nach mindestens einem der vorhergehenden Ansprüche, **dadurch gekennzeichnet, dass** die kosmetische Zubereitung ein oder mehrere Wirkstoffe enthält.

9. Kosmetische Zubereitung nach mindestens einem der vorhergehenden Ansprüche, **dadurch gekennzeichnet, dass** die kosmetische Zubereitung 0,5-5 Gew.-% DHA und/oder 0,1-5% Gew.-% Vitamin C (Ascorbinsäure) und/oder 0,1-5 Gew-% wässriger Anisfruchtextrakt (Bioxilift) und/oder 0,1-1 Gew.-% Creatin und/oder 0,001 -1 % Gew.-% an pflanzlichen Polyphenolen (z.B. Flavoide, AGR, Catechine (Tees)) und/oder 0,001-1% Gew.-% Licocalcone und/oder 0,001 - 0,3 Gew.-% Folsäure und/oder 0,001-0,5 Gew.-% Q10 enthalten, bezogen auf das Gesamtgewicht der Zubereitung, enthält.

10. Kosmetische Zubereitung nach mindestens einem der vorhergehenden Ansprüche, **dadurch gekennzeichnet, dass** die kosmetische Zubereitung bis zu 25 Vol.-% an Sauerstoff enthält, bezogen auf das Gesamtvolumen an kosmetischer Zubereitung.

11. Kosmetische Zubereitung nach mindestens einem der vorhergehenden Ansprüche, **dadurch gekennzeichnet, dass** die kosmetische Zubereitung keine Konservierungsstoffe enthält.

12. Zusätzliche Komponenten in Form von Tabletten oder Kapseln, zur Herstellung einer kosmetischen Zubereitung gemäß den Ansprüchen 1 bis 11.

13. Zusätzliche Komponenten in Form von Tabletten oder Kapseln nach Anspruch 12, **dadurch gekennzeichnet, dass** der oder die Wirkstoffe gewählt werden aus der Gruppe
- Antioxidantien, insbesondere Ubichinon Q10,
- Pflanzenextrakte, wie zum Beispiel Aloe Vera,
- körpereigenen Polymere, wie zum Beispiel Hyaluronsäure, Creatin,
- Vitamine, wie zum Beispiel Vitamin C oder E, Folsäure
- Hautaufheller (Whitening-Wirkstoffe), wie zum Beispiel Dioic Acid,
- selbstbräunende Wirkstoffe (Tannin-Wirkstoffe), wie zum Beispiel Dihydroxyaceton (DHA),
- Lichtschutzfilter, wie zum Beispiel Trianzine, Hydroxyzimtsäureester, Metalloxide.
